Europäisches Patentamt

European Patent Office

Office européen des brevets

(11) Veröffentlichungsnummer: **0 416 470 A2**

# EUROPÄISCHE PATENTANMELDUNG

(21) Anmeldenummer: 90116693.4

(22) Anmeldetag: 30.08.90

(51) Int. Cl.⁵: **B01D 71/10, C12P 19/04**

(30) Priorität: 04.09.89 DE 3929369

(43) Veröffentlichungstag der Anmeldung:
**13.03.91 Patentblatt 91/11**

(84) Benannte Vertragsstaaten:
**AT BE CH DE DK FR GB IT LI NL SE**

(71) Anmelder: **FRAUNHOFER-GESELLSCHAFT ZUR FÖRDERUNG DER ANGEWANDTEN FORSCHUNG E.V.**
**Leonrodstrasse 54**
**W-8000 München 19(DE)**

(72) Erfinder: **Gröbe, Anneliese**
**Filderstrasse 64**
**W-7300 Esslingen(DE)**
Erfinder: **Chmiel, Horst**
**Paracelsusstrasse 14**
**W-7250 Leonberg(DE)**
Erfinder: **Strathmann, Heinrich**
**Milanweg 15**
**W-7400 Tübingen(DE)**

(54) Verfahren zur Herstellung von Cellulose-Membranen aus bakteriell erzeugter Cellulose.

(57) Die Erfindung betrifft ein Verfahren zur Herstellung von Membranen aus bakteriell erzeugter Cellulose, sowie die Verwendung dieser Membranen für Trennoperationen, wie z.B. für die Mikrofiltration oder für die Ultrafiltration. Man läßt Cellulose bildende Essigsäure-Bakterien bei Temperaturen zwischen 18 und 35 °C emers in Standkulturen bei pH-Werten zwischen 5.5 und 6.5 auf Nährmedien wachsen, welche für die Bakterien verwertbare Kohlenstoff-Quellen, verwertbare Proteine und Nährsalze enthalten, man entfernt und verwirft die eventuell während einer exponentiellen Wachstumsphase der Bakterien von den Bakterien auf den Nährmedien gebildeten, morphologisch uneinheitlichen Cellulose-Schichten, und man isoliert und reinigt die während der stationären Wachstumsphase der Bakterien von den Bakterien auf den Nährmedien gebildeten morphologisch einheitlichen Cellulose-Schichten. In Abhängigkeit vom Nahrungsangebot für die Bakterien erhält man Cellulose-Membranen mit jeweils einheitlicher fibrillärer bzw. globulärer Struktur. Bei konstantem Nahrungsangebot erhält man erhält man Cellulose-Membranen mit einheitlicher Schichtdicke.

EP 0 416 470 A2

# VERFAHREN ZUR HERSTELLUNG VON CELLULOSE-MEMBRANEN AUS BAKTERIELL ERZEUGTER CELLU-LOSE

Die Erfindung betrifft ein Verfahren zur Herstellung von Cellulose-Membranen aus bakteriell erzeugter Cellulose, sowie die Verwendung dieser Membranen für Trennoperationen.

Cellulose-Membranen besitzen aufgrund ihrer Materialeigenschaften ein breites Einsatzspektrum. Eines ihrer Anwendungsgebiete liegt auf dem Gebiet der Membrantrennverfahren.

Filtrationsmembranen aus Cellulose werden z.B. dadurch hergestellt, daß Cellulose zunächst in lösliche Cellulosederivate, z.B. in Nitrocellulose übergeführt und gelöst wird, und daß diese Lösungen (Polymerlösungen) dann nach einem Phaseninversionsprozeß zu Flachmembranen verformt werden, indem z.B. auf einer Glasplatte oder auf einem Vlies diese Polymerlösungen mit einer Rakel zu einem Film ausgezogen werden. Lösungsmittel und Fällungsmittel werden ausgewaschen, die Membranen werden konditioniert und getrocknet. Damit sind sie lagerfähig und jederzeit verfügbar. Die Zusammensetzung und die Konzentration der Polymerlösung sowie die Fällbedingungen bestimmen die Membranstruktur. Die Membranstärke und damit die mechanische Stabilität und der transmembrane Fluß werden durch die Konzentration der Polymerlösung und durch die Rakelhöhe festgelegt. Je geringer die Membranstärke ist, desto besser ist in der Regel der transmembrane Fluß.

Die Membranstärke ist bei diesem Verfahren jedoch nicht beliebig herabsetzbar, z. B. durch eine Verringerung der Polymerkonzentration, denn mit abnehmender Polymerkonzentration sinken deren Filmbildungseigenschaften, d.h. die Polymerlösung reißt beim Rakeln ab. Auf diesem Wege sind Cellulose-Membranen mit Stärken unter 1 $\mu$m und ausreichender mechanischer Stabilität bei gleichzeitig hohem Wasserfluß nicht herstellbar.

Cellulose kommt z.B. in der Baumwolle, in Nadel- und Laubbaumen sowie in Einjahrespflanzen vor, allerdings nur im Verbund mit hauptsächlich Lignin und Hemicellulosen, die etwa 50 % ausmachen. Die Abtrennung der Cellulose aus dem Verbundwerkstoff Holz erfolgt nach den bekannten und in großem Maßstab durchgeführten Holzaufschlüssen. Vor allem aus ökologischer Sicht stellen die abgetrennten Produkte Lignin und Hemicellulose ein großes Problem dar. Der so gewonnene Zellstoff hat einen Durchschnittspolymerisationsgrad von 400-700.

Im Gegensatz hierzu wird durch cellulosebildende Bakterien, z.B. durch Acetobacter xylinum oder durch Acetobacter aceti in geeigneten Nährmedien eine reine Bakteriencellulose synthetisiert, die eine andere Morphologie und daher auch ein anderes Eigenschaftsprofil aufweist. Der durchschnittliche Polymerisationsgrad dieser Bakteriencellulose liegt mit DP = 2 000 dreimal höher als der des Holzzellstoffes.

Noch gravierender ist der Unterschied in der Kristallinität: Holzzellstoff ist nur zu etwa 80 % kristallin, Bakteriencellulose mit einem Kristallinitätsgrad von 0.96 ist praktisch zu über 90 % kristallin und kann daher Einkristalle bilden. Diese hohe Kristallinität äußert sich im strukturell unterschiedlichen Aufbau der Gitterzellen.

Für die bakterielle Synthese von Cellulose sind neben Sauerstoff als Nahrungsquelle Kohlenhydrate notwendig. Bevorzugt werden hierfür Hexosen. Eine der gebräuchlichsten Nährlösungen für Acetobacter xylinum nach Hestrin und Schramm (Biochem. Journal 58 , 345 (1954)) enthält 2 % Glucose, 0.5 % Petone, 0.5 % Hefeextrakt, 0.27 % NaH$_2$PO$_4$ und 0.16 % Zitronensäure (Monohydrate) und wird mit NaOH oder HCl auf einen pH-Wert von 6 eingestellt. Das Wachstum erfolgt bei 24 bis 30° C emers in Standkulturen, im Gegensatz zu anderen bekannten mikrobiellen Prozessen, die in Schüttelkulturen submers verlaufen. Die eigentliche Synthese der Bakteriencellulose erfolgt mit Hilfe eines polymerisationsauslösenden Multienzymkomplexes, wobei zunächst innerhalb des Bakteriums amorphe Cellulose gebildet wird, die dann durch sog. Extrusionsporen, die sich in der Membran des Bakteriums befinden, ausgeschieden wird. Dabei erfolgt der eigentliche morphologische Aufbau, indem "Häutchen" zusammengefaltet und zu einer fibrillaren Struktur umgeformt werden, die dann Bakteriencellulose-Vliese aufbauende "Cellulosestränge" bilden. Makroskopisch nimmt der Wachstumsprozeß folgenden Verlauf: Nach einer Wachstumszeit von etwa 24 Stunden ist in der Nährlösung eine leichte Trübung erkennbar, die aber bereits aus einem zusammenhängenden, hochgequollenen fibrillären Netzwerk besteht, das bis 2000 % Wasser, bezogen auf die Trockensubstanz, bindet. In diesem hochgequollenen Gel werden innerhalb etwa weiterer 24 Stunden inhomogene, inselartige Wachstumszentren sichtbar. Im Verlauf der nächsten 24 Stunden erfolgt eine Verdichtung, wobei ein Teil des vom Gel gebundenen Wassers abgegeben wird (Synärese) und das Vlies sich an der Oberfläche der Lösung als hochgequollene Masse abscheidet.

Die bei der Synthese zunächst als sog. Vlies anfallende Bakteriencellulose besitzt eine außerordentlich hohe Naßreißfestigkeit und widersteht mehrfacher Druckbelastung von 10 bar.

Da sich Bakteriencellulosen vorzüglich als Trennmaterialien eignen würden, wurde bereits versucht, derartig native Cellulose-Vliese als Osmometer-Membranen einzusetzen (Vgl. C.R. Masson, R.F. Menzies, J. Cruickshank, H.W. Melville, Nature 157 , S. 74, 1946). Diese Versuche scheiterten jedoch, da es auch unter optimierten Bedingungen des Wachstumsprozesses nicht möglich war, die für Trennprozesse notwendigen, morphologisch homogenen Strukturen herzustellen.

Da alle bisherigen Versuche, diese Uneinheitlichkeit während des Wachstumsprozesses zu beheben, fehlgeschlagen sind, wurden deshalb versucht, Membranen aus Bakteriencellulose nach einem technologisch aufwendigen Verfahren herzustellen. Dazu werden die bakteriell gewachsenen, morphologischen Strukturen der nativen Bakteriencellulose-Vliesmembranen zunächst zerstört, die Vliese werden aufgeschlagen, und anschließend werden daraus mit einem Blattbildungsverfahren erneut Membranen aufgebaut (DD-PS 92136). Diese Membranen bestehen zwar nun auch aus Bakteriencellulose, deren Struktur ist aber mit der ursprünglichen Struktur der nativen Bakteriencellulose-Vliesmembranen nicht mehr identisch.

Der Erfindung liegt daher die Aufgabe zugrunde, ein Verfahren bereit zustellen, mit welchem mechanisch stabile Cellulose-Membranen mit morphologisch homogenen Strukturen und gleichmäßiger Porengröße, d.h. mit einer engen Porenradienverteilung, und mit hohen transmembranen Flüssen einfach, schnell und universell hergestellt werden können. Diese Cellulose-Membranen sollen für einen Einsatz bei Trennverfahren von Gasen, Dämpfen und Flüssigkeiten geeignet sein.

Gelöst wird diese Aufgabe dadurch, daß man Cellulose bildende Essigsäure-Bakterien bei Temperaturen zwischen 18 und 35 °C emers in Standkulturen bei pH-Werten zwischen 5.5 und 6.5 auf Nährmedien wachsen läßt, welche für die Bakterien verwertbare Kohlenstoff-Quellen, verwertbare Proteine und Nährsalze enthalten, daß man die eventuell während einer exponentiellen Wachstumsphase der Bakterien von den Bakterien auf den Nährmedien gebildete(n), morphologisch uneinheitliche(n) Cellulose-Schicht(en) entfernt und verwirft, und daß man die während der stationären Wachstumsphase der Bakterien von den Bakterien auf den Nährmedien gebildeten, morphologisch einheitlichen Cellulose-Schichten isoliert und reinigt. Diese morphologisch einheitlichen Cellulose-Schichten können als Cellulose-Membranen in Membran-Trennverfahren eingesetzt werden.

Überraschenderweise wurde festgestellt, daß durch einen gezielten Eingriff in den Wachstumsprozeß der Cellulose bildenden Bakterien morphologisch völlig einheitliche Bakteriencellulose-Vliese erhalten werden können, die sogar noch bei sehr geringen Schichtdicken von z.B. nur 250 μm morphologisch völlig homogen sind und sogar bei diesen geringen Schichtdicken über eine ausgezeichnete mechanische Stabilität und über eine außerordentlich hohe Naßreißfestigkeit verfügen. Hierzu muß man die sich eventuell anfänglich während einer exponentiellen Wachstumsphase der Bakterien bildenden, inhomogenen Cellulose-Schichten von der Oberfläche des Nährmediums mechanisch entfernen. Diese Schichten sind aufgrund ihrer Inhomogenität für Trennverfahren nur bedingt geeignet. Die sich anschließend während der stationären Wachstumsphase der Bakterien bildenden Cellulose-Schichten sind völlig homogen und weisen die für Trennmembranen erforderlichen morphologisch einheitlichen Strukturen auf. In Abhängigkeit von den Wachstumsbedingungen für die Bakterien kann es erforderlich sein, eine oder mehrere inhomogene Cellulose-Schichten zu entfernen, bevor die Bakterien die stationäre Wachstumsphase erreichen und mit der Produktion der homogenen Cellulose-Schichten beginnen.

Als Bakterienkulturen zur Durchführung des erfindungsgemäßen Verfahrens können alle Cellulose bildenden Essigsäure-Bakterien eingesetzt werden, wie z.B. Acetobacter aceti oder Acetobacter xylinum. Als Nahrungsquelle können die üblichen Nährlösungen für Acetobacter-Stamme verwendet werden, die eine für die Bakterien verwertbare Kohlenstoff-Quelle erhalten, sowie verwertbare Proteine und Nährsalze, und deren pH-Wert zwischen 5.5 und 6.5 liegt. Bevorzugte Ausführungsformen des erfindungsgemäßen Verfahrens verwenden Nährlösungen, die einen pH-Wert von 6 besitzen. Geeignete Nährlösungen zur Durchführung des erfindungsgemäßen Verfahrens sind z.B. solche nach Hestrin und Schramm (Biochem. Journal 58 , 345 (1954)). Eine geeignete Nährlösung enthält z.B. 2 % Glucose, 0.5 % Petone, 0.5 % Hefeextrakt, 0.27 % $NaH_2PO_4$ und 0.16 % Zitronensäure (Monohydrate) und ist mit NaOH oder HCl auf einen pH-Wert von 6 eingestellt. Das Wachstum der Cellulose-Vliese erfolgt bei Temperaturen zwischen 18 und 35 °C, bevorzugt bei Temperaturen zwischen 24 bis 30 °C, emers in Standkulturen. Innerhalb von 24 bis 48 Stunden können die Vliese geerntet werden. In Figur 1 ist das emerse Wachstum der bakteriellen Cellulose-Membranen schematisch dargestellt

Überraschenderweise wurde festgestellt, daß man als Kohlenstoffquelle neben den bekannten Nährstoffen auch Abwasser der Lebensmittelindustrie, z.B. der Bier-, der Hefe-, der Milch- oder der Stärkeindustrie, der Zellstoff- oder der Cellulosefaserindustrie einsetzen kann. Damit ist eine Möglichkeit gegeben, die Abwässer dieser Industrien zu entsorgen.

Daß die sich während der exponentiellen Wachstumsphase der Bakterien bildenden ersten Cellulose-Schichten morphologisch uneinheitlich ausgebildet sind, ist darauf zurückzuführen, daß sich zunächst noch keine ausreichende Anzahl von Bakterien an der Oberfläche der Nährlösung angesammelt hat, also keine

3

einheitliche Matrize entstehen kann. Auch wenn man diese 1. Schicht beliebig dick wachsen läßt, entsteht kein einheitliches homogenes Vlies. Figur 2 zeigt die lichtmikroskopische Aufnahme einer inhomogenen, während der exponentiellen Wachstumsphase der Bakterien gebildeten Cellulose-Schicht.

Überraschenderweise wurde festgestellt, daß bereits die erste von den Bakterien gebildete Cellulose-Schicht völlig homogen und für einen Einsatz bei Trennverfahren geeignet ist, wenn man von einem genügend hohen Bakterientiter ausgeht, so daß sofort die stationäre Wachstumsphase der Bakterien erreicht wird.

Die nach dem erfindungsgemäßen Verfahren hergestellten homogenen Bakteriencellulose-Vliese sind in idealer Weise als Membranen für Trennoperationen geeignet, müssen aber vor ihrem Einsatz gereinigt werden, um restliche Bakterien zu zerstören und zu entfernen. Diese Reinigung kann z.B. durch Behandeln mit einer Base erfolgen. Anschließend werden die Cellulose-Schichten mit einer Säure neutralisiert und mit entionisiertem Wasser gewaschen. Bei bevorzugten Ausführungsformen des erfindungsgemäßen Verfahren werden die Cellulose-Membranen in bekannter Weise von restlichen Bakterien befreit, in dem sie z.B. in Laugen erhitzt werden, z.B. in 10 %-iger Natronlauge bei 50 °C, und anschließend mit Essigsäure neutralisiert werden. Sie werden mit entionisiertem Wasser gespült und können bereits in diesem Stadium, d.h. in feuchtem Zustand, für Trennoperationen eingesetzt werden.

Die nach dem erfindungsgemäßen Verfahren hergestellten Cellulose-Membranen können in der für Membranen üblichen Weise getrocknet werden und man erhält lagerfähige Cellulose-Membranen. Ferner ist es möglich, die nach dem erfindungsgemässen Verfahren hergestellten Cellulose-Membranen unter Verstreckung zu trocknen. Man erhält auf diese Weise besonders dauerhafte Cellulose-Membranen. Bei bevorzugten Ausführungsformen des erfindungsgemäßen Verfahrens werden Verstreckungsfaktoren zwischen 0.1 und 1.0 gewählt.

Die nach dem erfindungsgemäßen Verfahren hergestellten Cellulose-Membranen können in üblicher Weise konditioniert werden, z.B. mit Ethanol/Wasser-Gemischen in unterschiedlichen Mischungsverhältnissen, mit Methanol oder mit Cyclohexan.

Entfernt man die erste homogene Cellulose-Schicht von den Nährmedien und fügt man den Bakterien-Suspensionen keine zusätzlichen Nährstoffe zu, so sind alle nachwachsenden Cellulose-Schichten ebenfalls völlig homogen, die Schichtdicken sind jedoch infolge eines geringeren Nahrungsangebotes dünner, die Schichten aber noch einheitlicher. Überraschender Weise wurde festgestellt, daß sogar die dünnen, morphologisch einheitlichen Cellulose-Schichten mechanisch sehr stabil sind, - sie zeigen "bubble points" von > 9.5 bar -, dauerhaft ihre Struktur und ihre Gestalt behalten und nicht schrumpeln bzw. schrumpfen.

Die Bildung von Bakteriencellulose hört erst auf, wenn das Nahrungsangebot. z.B. die Glucose, verbraucht ist. Hält man das Nahrungsangebot dagegen konstant, dann resultieren Vliese mit vergleichbarer Schichtdicke.

Überraschenderweise wurde nun gefunden, daß in Abhängigkeit vom Nahrungsangebot während der stationären Wachstumsphase Cellulose-Vliese nicht nur mit unterschiedlichen Dicken. sondern auch mit unterschiedlichen, aber jeweils morphologisch einheitlichen Strukturen gebildet werden. die aufgrund dieser Unterschiede für unterschiedliche Einsatzzwecke geeignet sind. Bei einem konstanten Nahrungsüberangebot während der stationaren Wachstumsphase entstehen Cellulose-Vliese mit fibrillären Strukturen und mit Schichtdicken von 0.02 bis 2 mm. Diese Cellulose-Vliese eignen sich als Mikrofiltrationsmembranen. sie zeigen Wasserpermeabilitäten von 20 bis 20000 l·m²hbar und "bubble-points" zwischen 3 und 12 bar. Überraschenderweise wurde festgestellt, daß sich die Schichtdicken dieser Membranen aus Bakteriencellulose nach Entfernen des Druckes reversibel auf die ursprünglichen Werte einstellen. Die Figuren 3a, 3b und 3c zeigen rasterelektronenmikroskopische Aufnahmen einer erfindungsgemäßen Cellulose-Membranen mit fibrillärer Struktur. Figur 3a zeigt einen Schnitt und die Figuren 3b und 3c zeigen die Oberfläche.

Bei konstantem Nahrungsmangel während der stationären Wachstumsphase entstehen Cellulose-Vliese mit globulären Strukturen und mit extrem dünnen Schichtdicken von 200 bis 1000 nm. Diese Cellulose-Vliese zeigen Wasserpermeabilitäten von 2000 bis 15000 l·m²hbar und "bubble-points" zwischen 3 und 5 bar. Überraschenderweise wurde festgestellt, daß bei einer anschließenden Trocknung unter Verstreckung mit Faktoren von 0.1 bis 1 mechanisch besonders stabile. unbegrenzt lange lagerfähige. extrem dünne Cellulose-Membranen entstehen, die Substanzen bis zu Molgewichten von 12000 abtrennen. Die Figuren 4a, 4b und 4c zeigen rasterelektronenmikroskopische Aufnahmen einer erfindungsgemäßen Cellulose-Membran mit globulärer Struktur.

So werden z.B. bei Glucose-Konzentrationen von ca 2 % Cellulose-Membranen mit fibrillärer Struktur gebildet und bei Glucose-Konzentrationen von 0.05 % Cellulose-Membranen mit globulärer Struktur Unterhalb einer Glucose-Konzentration von 0.03 % hört die Membranbildung auf. Mit dem erfindungsgemäßen Verfahren können also wahlweise Mikro-oder Ultrafiltrationsmembranen hergestellt werden. Dicke Cellulose-Vliese ergeben Mikrofiltrationsmembranen und dünne Cellulose-Vliese ergeben unter Verstrek-

kung Ultrafiltrationsmembranen.

Überraschenderweise wurde festgestellt, daß man durch einen Wechsel zwischen Nahrungsüberangebot und Nahrungsmangel während der stationären Wachstumsphase kombinierte Mikro-und Ultrafiltrationsmembranen aus Bakteriencellulose erzeugen kann, die eine extrem dünne, aber eine geschlossene, dichte Oberflächentrennschicht, und darunter bzw. im Inneren wahlweise eine fibrilläre oder globuläre Struktur besitzen.

Anhand von Beispielen werden die Eigenschaften der nach dem erfindungsgemäßen Verfahren hergestellten Membranen näher aufgezeigt.

**Beispiel 1:**

| Abhängigkeit der Schichtdicken der geernteten Cellulose-Vliese vom abnehmenden Nahrungsangebot während der Wachstumsphasen der Cellulose-Schichten. | |
|---|---|
| Anzahl der Ernten | Schichtdicke (feucht) $\mu$m |
| 1 | 3 000 |
| 2 | 2 000 |
| 3 | 1 000 |
| 4 | 800 |
| 5 | 750 |
| 6 | 500 |
| 7 | 400 |
| 8 | 300 |
| 9 | 250 |

**Beispiel 2:**

Vergleich des "bubble-points" einer handelsüblichen Mikrofiltrationsmembran aus Cellulosemischester mit dem eines Bakteriencellulose-Vlieses, welches mit Natronlauge behandelt, mit Essigsäure neutralisiert und mit entionisiertem Wasser gewaschen wurde. Mit Hilfe des bubble-point-Tests nach ASTM F 316 erfolgt eine Charakterisierung der Membranen bezüglich Größe und Verteilung der Poren.

|   |   |   | Druck (bar) | Zahl d. Blasen |
|---|---|---|---|---|
| A | Bakteriencellulose-Vlies, wasserfeucht Membranstärke: 700 μm |   | 2.0 2.6 4.8 6.0 8.0 | 1 2 2 2 3 |
|   |   | bubble-point | > 9.5 | ∞ |
| B | handelsübl. Mikrofiltrationsmembran aus Cellulosemischester, mittl. Porengröße 0.1 μm wasserfeucht Membranstärke 110 μm |   | 2.0 2.6 4.8 6.0 8.0 9.5 | 0 0 0 0 0 1 |
|   |   | bubble-point | > 9.5 | ∞ |

Hieraus ergibt sich, daß sich das Bakteriencellulose-Vlies wie eine Mikrofiltrationsmembran verhält, daß die mittlere Porengröße um 0.1 μm liegen muß, da die handelsübliche Membran eine mittlere Porengröße von 0.1 μm besitzt und daß die mittlere Porengröße sehr einheitlich ist, denn der Durchtritt der Luft durch die Membran erfolgt schlagartig.

**Beispiel 3:**

Eine Überprüfung des Porensystems mit Latexkugeln in einer cross-flow Zelle ergab folgende Werte:

|   | Durchmesser Latexkugeln (μm) | Rückhaltevermögen (%) |
|---|---|---|
| Bakterien-Cellulosemembran | 0.093 0.3 | 94-97 100 |
| handelsübliche Mikrofiltrationsmembran Cellulosemischester, mittlere Porengröße 0.1 μm | 0.093 0.3 | 97 100 |

Aus der Messung des "bubble-point" und des Rückhaltevermögens für Latexkugeln ergibt sich für die wasserfeuchten Bakteriencellulose-Membranen eine mittlere Porengröße von 0.1 μm.

**Beispiel 4:**

| Probe | | Druck (bar) | Permeabilität (1/m²h bar) |
|---|---|---|---|
| A/1 | Bakteriencellulosemembran wasserfeucht<br>Membranstärke: 700 μm | 0.1<br>0.2<br>0.4 | 1212<br>1267<br>1112 |
| A/2 | nach 2 h Betriebsdauer mit 0.4 bar erneut vermessen | 0.1<br>0.2<br>0.4 | 749<br>725<br>614 |
| | Membranstärke nach Beendigung des Versuches : 300 μm | | |
| A/3 | Membran 24 h bei Normaldruck außerhalb der<br>Meßzelle in bidest. Wasser gelagert Membranstärke:<br>700 μm | 0.1<br>0.2<br>0.4<br>0.8 | 1400<br>1430<br>1315<br>1017 |
| B/1 | Handelsübl. Mikrofiltr.-M. aus Cellulosemischester mittl.<br>Porengröße: 0.1 μm | 0.1<br>0.2<br>0.4 | 403<br>352<br>110 |
| | Membranstärke : 110 μm | 1.0 | 39 |
| B/2 | Membran 24 h bei Normaldruck außerhalb der<br>Meßzelle in bidest. Wasser gelagert Membranstärke<br>100 μm | 0.1<br>0.2<br>0.5<br>1.0 | 44<br>37<br>30<br>22 |

Von besonderen anwendungstechnischen Vorteil ist die hohe Elastizität der Bakteriencellulose-Membranen im Vergleich zu Cellulosemischester-Membranen. Unter Druckbelastung reduziert sich zwar die Membranstärke unter den hier angewandten Bedingungen etwa auf die Hälfte, nach einer willkürlich gewählten Erholungsphase von 24 Stunden stellen sich sowohl die ursprünglichen Membranstärken als auch die alten bubble-point Werte wieder ein.

Daraus ergeben sich die folgenden Vorteile der erfindungsgemässen Membranen. Die Bakteriencellulose-Membranen weisen im Vergleich mit den Cellulosemischester-Membranen trotz gleicher Porengröße eine um den Faktor 3 erhöhte Permeabilität auf. Bei Druckentlastung verhält sich die Bakteriencellulose-Membran reversibel, d. h. der alte Wasserfluß stellt sich wieder ein.

Damit ist gezeigt, daß die nach dem erfindungsgemäßen Verfahren aus Bakteriencellulose hergestellten Cellulose-Membranen sich in hervorragender Weise für den Einsatz in Membranentrennverfahren eignen.

**Beispiel 5:**

Die Bestimmung der transmembranen Flüsse erfindungsgemäßer, feuchter Cellulose-Membranen mit verschiedenen Schichtdicken und konstanten Porengrößen von weniger als 0.1 μm lieferte die folgende Werte.

| Schichtdicke [μm] | transmembraner Fluß [1/m²h bar] |
|---|---|
| 100 | 2976.73 |
| 200 | 2716.27 |
| 400 | 930.23 |
| 700 | 511.62 |

**Beispiel 6:**

Es wurde jeweils der transmembrane Fluß einer handelsüblichen Mikrofiltrationsmembran aus Cellulosemischester (a) mit einer mittleren Porengröße von 0.1 µm, einer erfindungsgemäßen, getrockneten und modifizierten Cellulose-Membran (b) und einer feuchten, erfindungsgemäßen Cellulose-Membran (c) mit einer Schichtdicke von 100 µm und mit einer Porengröße von < 0.1 µm in Abhängigkeit von der Zeit bestimmt. Figur 5 zeigt die Ergebnisse in Form eines Diagrammes. Die Meßergebnisse zeigen sehr eindrucksvoll, daß die Permeabilität der handelsüblichen Celluose-Membran im Vergleich zur feuchten, erfindungsgemäßen Membran bei vergleichbarer Porengröße wesentlich geringer ist, und daß bei der handelsüblichen Celluose-Membran im Vergleich zur feuchten, erfindungsgemäßen Membran im Laufe der Zeit eine wesentlich stärkere Abnahme des transmembranen Flusses zu verzeichnen war.

**Beispiel 7:**

Titerbestimmung von Acetobacter Xylinum in Abhängigkeit von der Zahl der Ernten der Cellulose-Membranen.

In einem Fernbachkolben wurden jeweils 300 ml einer Suspension des Bakteriums Acetobacter Xylinium in einer Nährlösung nach Hestrin und Schramm (2 % Glucose, 0.5 % Petone, 0.5 % Hefeextrakt, 0.27 % $NaH_2PO_4$, 0.16 % Zitronensäure (Monohydrate), pH-Wert = 6) eingesetzt. Die Konzentration der Suspension betrug 1.0 x $10^6$ Bakterien pro ml. Der Fernbachkolben wurde bei 26 °C unter sterilen Bedingungen gelagert. Im Abstand von 2 Tagen wurden die von den Bakterien produzierten Cellulose-Membranen von den Suspensionen abgeerntet. Dazu wurden die Membranen jeweils mit einem sterilen Metallsieb von den Suspensionen getrennt. Die geernteten Membranen wurden zur Bestimmung ihrer Struktur aufgearbeitet. Jeweils ein Teil der Bakteriensuspensionen wurde für die Konzentrationsbestimmung verwendet. Vom verbleibenden Teil der Bakteriensuspensionen wurde die Menge bestimmt, die wieder in den Fernbachkolben übergeführt wurde. Auf diese Weise wurden jeweils 11 Membranen geerntet und jeweils zum Zeitpunkt der Ernte wurden die Konzentrationen der Bakterien bestimmt. Die nachfolgenden Tabellen zeigen die Meßergebnisse von zwei Versuchsreihen. Aufgrund von rasterelektronenmikroskopischen Aufnahmen wurden sowohl Cellulose-Membranen mit fibrillärer als auch mit globulärer Struktur isoliert. Mit zunehmender Anzahl der Ernten nahm die Glucose-Konzentration von einem Ausgangswert von 2 % auf 0.05 % ab, wobei bei "hohen" Glucose-Konzentrationen Cellulose-Membranen mit fibrillärer Struktur isoliert wurden und bei "niedrigen" Glucose-Konzentrationen Membranen mit globulärer Struktur.

| Datum | Uhrzeit | t[h][a] | V[ml][b] | Konz.[c] | Anzahl[d] | Ernte |
|---|---|---|---|---|---|---|
| 22.6 | $10^{40}$ | -- | 300 | $1.0 \times 10^6$ | $3.0 \times 10^8$ | - |
| 24.6. | $15^{10}$ | 52.5 | 250 | $2.3 \times 10^7$ | $5.75 \times 10^9$ | 1 |
| 26.6. | $11^{45}$ | 44.5 | 240 | $1.3 \times 10^8$ | $3.12 \times 10^{10}$ | 2 |
| 28.6. | $15^{00}$ | 51.0 | 210 | $1.3 \times 10^8$ | $2.73 \times 10^{10}$ | 3 |
| 30.6 | $9^{30}$ | 42.5 | 180 | $1.3 \times 10^8$ | $2.34 \times 10^{10}$ | 4 |
| 2.7. | $14^{30}$ | 53.0 | 160 | $4.4 \times 10^7$ | $7.04 \times 10^9$ | 5 |
| 4.7 | $11^{00}$ | 44.5 | 140 | $1.1 \times 10^8$ | $1.54 \times 10^{10}$ | 6 |
| 6.7. | $13^{50}$ | 51.0 | 120 | $2.6 \times 10^8$ | $3.12 \times 10^{10}$ | 7 |
| 8.7 | $14^{30}$ | 48.5 | 100 | $2.1 \times 10^{10}$ | $2.10 \times 10^{12}$ | 8 |
| 10.7 | $17^{10}$ | 50.5 | 83 | $3.2 \times 10^9$ | $2.66 \times 10^{11}$ | 9 |
| 12.7. | $15^{45}$ | 46.5 | 78 | $1.4 \times 10^9$ | $1.05 \times 10^{11}$ | 10 |
| 14.7 | $16^{30}$ | 48.5 | 76 | $1.2 \times 10^9$ | $9.12 \times 10^{10}$ | 11 |
| 22.6. | $10^{40}$ | -- | 300 | $2.8 \times 10^6$ | $8.46 \times 10^8$ | - |
| 24.6. | $15^{10}$ | 52.5 | 250 | $2.7 \times 10^7$ | $6.75 \times 10^9$ | 1 |
| 26.6. | $11^{45}$ | 44.5 | 240 | $2.1 \times 10^8$ | $5.04 \times 10^{10}$ | 2 |
| 28.6. | $15^{00}$ | 51.0 | 210 | $6.1 \times 10^7$ | $1.29 \times 10^{10}$ | 3 |
| 30.6. | $9^{30}$ | 42.5 | 190 | $1.0 \times 10^8$ | $1.90 \times 10^{10}$ | 4 |
| 2.7. | $14^{30}$ | 53.0 | 170 | $4.9 \times 10^7$ | $8.33 \times 10^9$ | 5 |
| 4.7. | $11^{00}$ | 44.5 | 152 | $9.0 \times 10^7$ | $1.37 \times 10^{10}$ | 6 |
| 6.7. | $13^{50}$ | 51.0 | 134 | $3.2 \times 10^8$ | $4.29 \times 10^{10}$ | 7 |
| 8.7. | $14^{30}$ | 48.5 | 110 | $9.2 \times 10^9$ | $1.01 \times 10^{12}$ | 8 |
| 10.7. | $17^{10}$ | 50.5 | 59 | $3.0 \times 10^8$ | $1.77 \times 10^{10}$ | 9 |
| 12.7. | $15^{45}$ | 46.5 | 45 | $8.6 \times 10^8$ | $3.87 \times 10^{10}$ | 10 |
| 14.7. | $16^{30}$ | 48.5 | 22.5 | $1.1 \times 10^9$ | $2.48 \times 10^{10}$ | 11 |

a: Wachstumszeit der Cellulose-Membranen zwischen den Ernten

b: Volumen der Bakteriensuspension

c: Bakterienkonzentration [.ml]

d: Bakterienzahl

## Beispiel 8:

Titerbestimmung von Acetobacter Xylinum in Abhängigkeit von der Zahl der Ernten der Cellulose-Membranen. Die Versuchsdurchführung und die Titerbestimmung erfolgte wie im Beispiel 7 beschrieben, das Ausgangsvolumen der Bakteriensuspension betrug 500 ml, die Anfangskonzentration war $5.4 \times 10^4$ bzw. $7.1 \times 10^4$ Bakterien pro ml und es wurden 12 Ernten eingeholt. Die nachfolgenden Tabellen zeigen die Meßergebnisse von zwei Versuchsreihen. Aufgrund von rasterelektronenmikroskopischen Aufnahmen wurden wie bei Beispiel 7 sowohl Cellulose-Membranen mit fibrillärer als auch mit globulärer Struktur isoliert. Mit zunehmender Anzahl der Ernten nahm die Glucose-Konzentration wieder von einem Ausgangswert von 2 % auf 0.05 % ab, wobei bei "hohen" Glucose-Konzentrationen Cellulose-Membranen mit fibrillärer Struktur isoliert wurden und bei "niedrigen" Glucose-Konzentrationen Membranen mit globulärer Struktur.

| Datum | Uhrzeit | t[h]$^a$ | V[ml]$^b$ | Konz.$^c$ | Anzahl$^d$ | Ernte |
|---|---|---|---|---|---|---|
| 2.7. | 14$^{30}$ | -- | 500 | 5.4x10$^4$ | 2.7 x10$^7$ | - |
| 4.7. | 11$^{00}$ | 44.5 | 426 | 1.3x10$^7$ | 5.54x10$^9$ | 1 |
| 6.7. | 13$^{50}$ | 51.0 | 414 | 1.4x10$^7$ | 5.59x10$^9$ | 2 |
| 8.7. | 14$^{30}$ | 48.5 | 376 | 6.0x10$^6$ | 2.26x10$^9$ | 3 |
| 10.7. | 17$^{00}$ | 50.5 | 342 | 6.4x10$^6$ | 2.19x10$^9$ | 4 |
| 12.7. | 15$^{45}$ | 47.0 | 320 | 5.6x10$^7$ | 1.79x10$^{10}$ | 5 |
| 14.7. | 16$^{15}$ | 48.5 | 303 | 3.9x10$^7$ | 1.18x10$^{10}$ | 6 |
| 16.7. | 14$^{15}$ | 46.0 | 243 | 2.6x10$^7$ | 6.32x10$^9$ | 7 |
| 18.7. | 14$^{45}$ | 48.5 | 200 | 3.2x10$^7$ | 6.40x10$^9$ | 8 |
| 20.7. | 12$^{00}$ | 45.0 | 153 | 2.6x10$^7$ | 3.98x10$^9$ | 9 |
| 22.7. | 16$^{00}$ | 52.5 | 148 | 4.4x10$^7$ | 6.51x10$^9$ | 10 |
| 24.7. | 16$^{40}$ | 48.5 | 135 | 5.1x10$^7$ | 6.89x10$^9$ | 11 |
| 26.7. | 14$^{30}$ | 46.0 | 120 | - | - | 12 |
| 2.7. | 14$^{30}$ | -- | 500 | 7.1x10$^4$ | 3.55x10$^7$ | - |
| 4.7. | 11$^{00}$ | 44.5 | 480 | 3.6x10$^7$ | 1.73x10$^{10}$ | 1 |
| 6.7. | 13$^{50}$ | 51.0 | 420 | 2.8x10$^7$ | 1.16x10$^{10}$ | 2 |
| 8.7. | 14$^{30}$ | 48.5 | 400 | 2.1x10$^7$ | 8.40x10$^9$ | 3 |
| 10.7. | 17$^{00}$ | 50.5 | 386 | 1.9x10$^7$ | 7.33x10$^9$ | 4 |
| 12.7. | 15$^{45}$ | 47.0 | 370 | 4.1x10$^7$ | 1.52x10$^{10}$ | 5 |
| 14.7. | 16$^{15}$ | 48.5 | 321 | 2.6x10$^7$ | 8.35x10$^9$ | 6 |
| 16.7. | 14$^{15}$ | 46.0 | 265 | 2.6x10$^7$ | 3.71x10$^9$ | 7 |
| 18.7. | 14$^{45}$ | 48.5 | 210 | - | - | 8 |
| 20.7. | 12$^{00}$ | 45.0 | 154 | 1.5x10$^7$ | 2.33x10$^9$ | 9 |
| 22.7. | 16$^{00}$ | 52.5 | 145 | 2.5x10$^7$ | 3.63x10$^9$ | 10 |
| 24.7. | 16$^{40}$ | 48.5 | 124 | 1.1x10$^7$ | 1.36x10$^9$ | 11 |
| 26.7. | 14$^{30}$ | 46.0 | 95 | - | - | 12 |

a: Wachstumszeit der Cellulose-Membranen zwischen den Ernten
b: Volumen der Bakteriensuspension
c: Bakterienkonzentration [.ml]
d: Bakterienzahl

Die Figuren 6 und 7 zeigen in Form von Diagrammen die Meßergebnisse zweier weiterer Titerbestimmungen von Acetobacter Xylinum in Nährlösungen nach Hestrin und Schramm. Die Bakterienkonzentrationen wurden jeweils zum Zeitpunkt der Ernte der Cellulose-Membranen bestimmt und aus dem Verlauf der Kurven ist deutlich erkennbar, daß die morphologisch einheitlichen Cellulose-Membranen während der stationären Wachstumsphase der Bakterien gebildet werden.

## Beschreibung der Figuren :

Figur 1 : zeigt schematisch das emerse Wachstum der bakteriellen Cellulose-Membranen in einem Fernbachkolben.

Figur 2 : zeigt die lichtmikroskopische Aufnahme einer inhomogenen, während der exponentiellen Wachstumsphase der Bakterien gebildeten Cellulose-Schicht.

Figur 3a: zeigt in einer rasterelektronenmikroskopischen Aufnahme den Schnitt einer erfindungsgemäßen Cellulose-Membran mit fibrillärer Struktur.

Figur 3b und Figur 3c: zeigen in rasterelektronenmikroskopischen Aufnahmen die Oberfläche einer erfindungsgemäßen Cellulose-Membran mit fibrillärer Struktur in verschiedenen Vergrößerungen.

Figur 4a, Figur 4b und Figur 4c: zeigen in rasterelektronenmikroskopische Aufnahmen die Oberfläche einer erfindungsgemäßen Cellulose-Membran mit globulärer Struktur in verschiedenen Vergrößerungen.

Figur 5 : zeigt die transmembranen Flüsse von

a) einer handelsüblichen Mikrofiltrationsmembran aus Cellulosemischester mit einer Porengröße von 0.1 μm,

b) einer getrockneten, modifizierten erfindungsgemäßen Membran und

c) einer feuchten erfindungsgemäßen Membran mit einer Schichtdicke von 100 μm und mit einer Porengröße von 0.1 μm.

Figur 6 und Figur 7 : zeigen Titerbestimmungen von Acetobacter Xylinum in Nährlösungen von Hestrin und Schramm jeweils zum Zeitpunkt der Ernte der Cellulose-Membranen.

## Ansprüche

1. Verfahren zur Herstellung von Membranen aus bakteriell erzeugter Cellulose, **dadurch gekennzeichnet** , daß man Cellulose bildende Essigsäure-Bakterien bei Temperaturen zwischen 18 und 35 °C emers in Standkulturen bei pH-Werten zwischen 5.5 und 6.5 auf Nährmedien wachsen läßt, welche für die Bakterien verwertbare Kohlenstoff-Quellen, verwertbare Proteine und Nährsalze enthalten, daß man die eventuell während einer exponentiellen Wachstumsphase der Bakterien von den Bakterien auf den Nährmedien gebildeten morphologisch uneinheitlichen Cellulose-Schichten entfernt und verwirft, und daß man die während der stationären Wachstumsphase der Bakterien von den Bakterien auf den Nährmedien gebildeten morphologisch einheitlichen Cellulose-Schichten ( = Cellulose-Membranen) isoliert und reinigt.

2. Verfahren nach Anspruch 1, **dadurch gekennzeichnet** , daß man von einem genügend hohen Bakterientiter ausgeht, so daß sofort die stationäre Wachstumsphase der Bakterien erreicht wird und bereits die erste von den Bakterien gebildete Cellulose-Schicht morphologisch einheitlich ist.

3. Verfahren nach Anspruch 1 oder 2, **dadurch gekennzeichnet** , daß man die morphologisch einheitlichen Cellulose-Schichten mit einer Base behandelt, um restliche Bakterien zu zerstören, daß man die Base mit einer Säure neutralisiert, und daß man die Cellulose-Schichten mit entionisiertem Wasser wäscht.

4. Verfahren nach Anspruch 3, **dadurch gekennzeichnet** , daß man die morphologisch einheitlichen Cellulose-Schichten mit verdünnter Natronlauge und mit Essigsäure behandelt.

5. Verfahren nach einem oder mehreren der Ansprüche 1 bis 4, **dadurch gekennzeichnet** , daß man die Bakterien während der Bildung der morphologisch einheitlichen Cellulose-Schichten einem konstanten Nahrungsangebot aussetzt.

6. Verfahren nach Anspruch 5, **dadurch gekennzeichnet** , daß man die Bakterien während der Bildung der morphologisch einheitlichen Cellulose-Schichten einem Nahrungsüberangebot aussetzt.

7. Verfahren nach Anspruch 5, **dadurch gekennzeichnet** , daß man die Bakterien während des Wachstums der morphologisch einheitlichen Cellulose-Schichten einem Nahrungsmangel aussetzt.

8. Verfahren nach einem oder mehreren der Ansprüche 1 bis 4, **dadurch gekennzeichnet** , daß man die Bakterien während des Wachstums der morphologisch einheitlichen Cellulose-Schichten abwechselnd einem Nahrungsüberangebot und einem Nahrungsmangel aussetzt.

9. Verfahren nach Anspruch 6, **dadurch gekennzeichnet** , daß man morphologisch einheitliche Cellulose-Schichten mit Schichtdicken von 0.02 bis 2 mm isoliert.

10. Verfahren nach Anspruch 7, **dadurch gekennzeichnet** , daß man morphologisch einheitliche Cellulose-Schichten mit Schichtdicken von 200 bis 1000 nm isoliert.

11. Verfahren nach einem oder mehreren der Ansprüche 1 bis 10, **dadurch gekennzeichnet** , daß man die morphologisch einheitlichen Cellulose-Schichten in der für Cellulose-Membranen üblichen Weise konditioniert.

12. Verfahren nach Anspruch 11, **dadurch gekennzeichnet** , daß man die morphologisch einheitlichen Cellulose-Schichten mit Ethanol Wasser-Gemischen behandelt.

13. Verfahren nach einem oder mehreren der Ansprüche 1 bis 12, **dadurch gekennzeichnet** , daß man die morphologisch einheitlichen Cellulose-Schichten in der für Membranen üblichen Weise trocknet.

14. Verfahren nach Anspruch 13, **dadurch gekennzeichnet** , daß man die morphologisch einheitlichen Cellulose-Schichten unter Verstreckung trocknet.

15. Verfahren nach Anspruch 14, **dadurch gekennzeichnet** , daß man eine Verstreckung um den Faktor 0.1 bis 1 durchführt.

16. Verfahren nach einem oder mehreren der Ansprüche 1 bis 15, **dadurch gekennzeichnet** , daß man als cellulosebildende Bakterien Stämme von Acetobacter aceti oder von Acetobacter xylinum einsetzt.

17. Verfahren nach einem oder mehreren der Ansprüche 1 bis 16, **dadurch gekennzeichnet** , daß man die Bakterien während des Wachstums der morphologisch einheitlichen Cellulose-Schichten bevorzugt bei Temperaturen von 24 bis 30 °C hält.

18. Verfahren nach einem oder mehreren der Ansprüche 1 bis 17, **dadurch gekennzeichnet** , daß man die Bakterien während des Wachstums der morphologisch einheitlichen Cellulose-Schichten bevorzugt bei einem pH-Wert von 6 hält.

19. Verfahren nach einem oder mehreren der Ansprüche 1 bis 18, **dadurch gekennzeichnet** , daß man als Kohlenstoffquelle für die Bakterien Abwässer der Lebensmittelindustrie, der Zellstoff- und/oder der Cellulosefaserindustrie einsetzt.

20. Verfahren nach Anspruch 19, **dadurch gekennzeichnet** , daß man Abwässer der Bier- und/oder der Hefe- und/oder der Milch- und/oder der Stärkeindustrie einsetzt.

21. Cellulose-Membranen, **gekennzeichnet durch** ihre Herstellung nach einem Verfahren nach einem oder mehreren der Ansprüche 1 bis 20.

22. Verwendung der Cellulose-Membranen nach Anspruch 21 für Trennprozesse.

23. Verwendung der Cellulose-Membranen nach Anspruch 21 für die Mikrofiltration oder für die Ultrafiltra-tion.